# EUROPEAN PATENT SPECIFICATION

(11) **EP 4 614 154 B1**
(45) Date of publication and mention of the grant of the patent: **02.12.2026**
(21) Application number: 25188763.4
(22) Date of filing: 15.10.2018
(51) Int. Cl.: G01N 33/50, G01N 33/569

(54) **GRP78-DERIVED PEPTIDE FOR IDENTIFYING HIGH-EFFICIENCY STEM CELLS**
PEPTIDE DÉRIVÉ DE GRP78 PERMETTANT L'IDENTIFICATION DE CELLULES SOUCHES À HAUT RENDEMENT
GRP78-ABGELEITETES PEPTID ZUR IDENTIFIZIERUNG HOCHWIRKSAMER STAMMZELLEN

(30) Priority: 13.10.2017 KR 20170133614
(43) Date of publication of application: 10.09.2025
(62) Document number(s) of the earlier application(s) in accordance with Art. 76 EPC: 18866139.1 / 3 696 547
(73) Proprietor: Nibec Co., Ltd., Chungcheongbuk-do 27816 (KR); Chungbuk National University Industry-Academic Cooperation Foundation, Cheongju-si, Chungcheongbuk-do 28644 (KR); Seoul National University R&DB Foundation, Seoul 08826 (KR)
(72) Inventor: PARK, Yoon Shin, 28415 Cheongju-si (KR); PARK, Yoon Jeong, 08291 Seoul (KR); CHUNG, Chong-Pyoung, 05618 Seoul (KR); LEE, Jue-Yeon, 13831 Gwacheon-si (KR)
(74) Representative: Kraus & Lederer PartGmbB

(56) References cited:
- CN-A- 101 445 551
- HUGHES SCOTT J. ET AL: "Probing the ATP Site of GRP78 with Nucleotide Triphosphate Analogs", vol. 11, no. 5, 4 May 2016 (2016-05-04), pages e0154862, XP055832278, Retrieved from the Internet <URL:https://storage.googleapis.com/plos-corpus-prod/10.1371/journal.pone.0154862/1/pone.0154862.pdf?X-Goog-Algorithm=GOOG4-RSA-SHA256&X-Goog-Credential=wombat-sa@plos-prod.iam.gserviceaccount.com/20210816/auto/storage/goog4_request&X-Goog-Date=20210816T130928Z&X-Goog-Expires=86400&X-Goog-SignedHeaders=h> DOI: 10.1371/journal.pone.0154862

## Description

### Technical Field

The present invention relates to a GRP78-derived peptide for screening highly efficient stem cells and the use thereof, and more particularly, to screening highly efficient stem cells using, as a marker, a GRP78-derived peptide capable of binding to the binding domain of GRP78 protein on the cell surface.

In detail, the present invention relates to a peptide for screening highly efficient stem cells, the peptide consisting of the amino acid sequence of SEQ ID NO: 8, as well as methods using the peptide and kits comprising the peptide.

### Background Art

In recent years, various studies have been conducted on the possibility of using stem cells as cell therapy by differentiating stem cells, such as embryonic stem cells and adult stem cells, into various cells. Multipotent embryonic stem cells have attracted attention as cell therapy products due to their ability to differentiate into various cells. However, the use of embryonic stem cells poses ethical problems, and hence it is difficult to use these embryonic stem cells as cell therapy in practice. To avoid such ethical problems, studies on the use of adult stem cells have been actively conducted *(*Trends in Neurosciences 23:450, 2000).

Although adult stem cells have disadvantages in that when these cells are transplanted into other persons, they carry the risk of infection and have a relatively low differentiation potential, these cells have advantages in that they can be obtained in large numbers and are very safe for medical applications. Specifically, even when these cells are transplanted into the body for organ regeneration, they do not cause cancer, and do not cause immune rejection because they have originated from the adult body. Thus, these adult stem cells can be used for autologous transplantation. In addition, adult stem cells have site-specific differentiation potential according to the characteristics of surrounding tissue, and do not cause cancer even when they are injected in an undifferentiated state. Thus, these adult stem cells advantageously have the potential to produce necessary cells immediately after transplantation and also have self-renewal potential to create and store undifferentiated stem cells, if necessary ( JACC: Basic to Translational Science 2:702-716, 2017).

During development of stem cells into a cell therapy product, a process of culturing isolated stem cells for a long period of time is essential in order to obtain the number of cells required to have an optimal therapeutic effect. Due to the long-term culture, development of the cell therapy product is inevitably performed in a state in which a large number of old cells is mixed or cells at various passages are mixed. This can cause problems in the stability and effectiveness of the stem cell therapy product, as well as quality control *(*European Cells and Materials 31: 136-159, 2016). Therefore, in order to develop a cell therapy product using adult stem cells, a process of screening old cells mixed due to long-term culture is necessary. Although various efforts have, in fact, been made to control the quality of stem cell therapy products, established clear markers are still insufficient.

Meanwhile, GRP78 is an HSP 70 family chaperone protein that is present in the ER lumen. GRP78 is an important factor in protein transport/transfer into the ER and is a functional protein that is a key to maintaining intracellular calcium homeostasis. This protein consists of two domains connected to each other: one is a nucleotide-binding domain (NBD) that binds and degrades nucleotides in the ER, more specifically ATP, and the other is a substrate-binding domain (SBD) that binds to metabolic enzymes such as ATPase, as well as metabolism-related proteins and metabolic substrates (e.g., ADP) *(*Gene 618:14-23, 2017). These domains regulate energy metabolism and calcium metabolism inside and outside the ER while degrading ATP depending on intracellular calcium and ATP concentrations during metabolism. CN101445551A discloses human BiP antigen epitope peptides (BiP is a synonym for GRP78) and discloses a variety of corresponding peptides differing in length from the peptide of the invention.

Accordingly, the present inventors have made extensive efforts to find markers which may be used to remove old stem cells and screen only highly efficient stem cells, and as a result, have found that the protein GRP78 associated with glucose metabolism can be used as a senescence-associated stem cell marker as the number of passages increases during long-term culture of adult stem cells, and also found that the effect of the GRP78 protein can be enhanced by introducing the GRP78 protein into cells, thereby completing the present invention.

The above information disclosed in this Background section is only for enhancement of understanding of the background of the present invention. Therefore, it may not contain information that forms a conventional art that is already known in the art to which the present invention pertains.

### Summary

An object of the present specification is to provide a peptide comprising a specific amino acid sequence capable of recognizing highly efficient stem cells, or a peptide capable of enhancing the effect of GRP78 protein, among the amino acid sequences of the marker GRP78 for developing a cell therapy product using adult stem cells and controlling the quality of the cell therapy product.

Another object of the present specification is to provide a method of screening highly efficient stem cells using the peptide or a method of increasing the efficiency of stem cells using the peptide to produce a stem cell therapy product having excellent efficacy.

To achieve the above objects, the present specification provides a peptide for screening highly efficient stem cells or increasing the efficiency of stem cells, the peptide being represented by an amino acid sequence selected from the group consisting of SEQ ID NOs: 1 to 18.

Also disclosed is a method of screening highly efficient stem cells using the peptide.

Disclosed but not claimed is a composition for screening highly efficient stem cells comprising the peptide.

The present invention also provides a kit for screening highly efficient stem cells comprising the peptide consisting of the amino acid sequence of SEQ ID NO: 8.

Disclosed but not claimed is a method of increasing the efficiency of stem cells by treating the stem cells with the peptide.

Disclosed but not claimed is a composition for increasing the efficiency of stem cells comprising the peptide. The following items are embodiments of the invention:
1. A peptide for screening highly efficient stem cells, the peptide consisting of the amino acid sequence of SEQ ID NO: 8. 2. The peptide of item 1, wherein the peptide is derived from GRP78. 3. The peptide of item 1, wherein the peptide recognizes ATP in the nucleus, which is regulated by GRP78. 4. The peptide of item 1, wherein the highly efficient stem cells express GRP78. 5. A method of screening highly efficient stem cells using the peptide of any one of items 1 to 4. 6. The method of item 5, comprising steps of: (a) conjugating a fluorescent to a peptide specific for GRP78; (b) binding the fluorescent-conjugated peptide to stem cells; and (c) screening GRP78-expressing stem cells under a fluorescence microscope. 7. A kit for screening highly efficient stem cells comprising the peptide of any one of items 1 to 4.

### Brief Description of Drawings

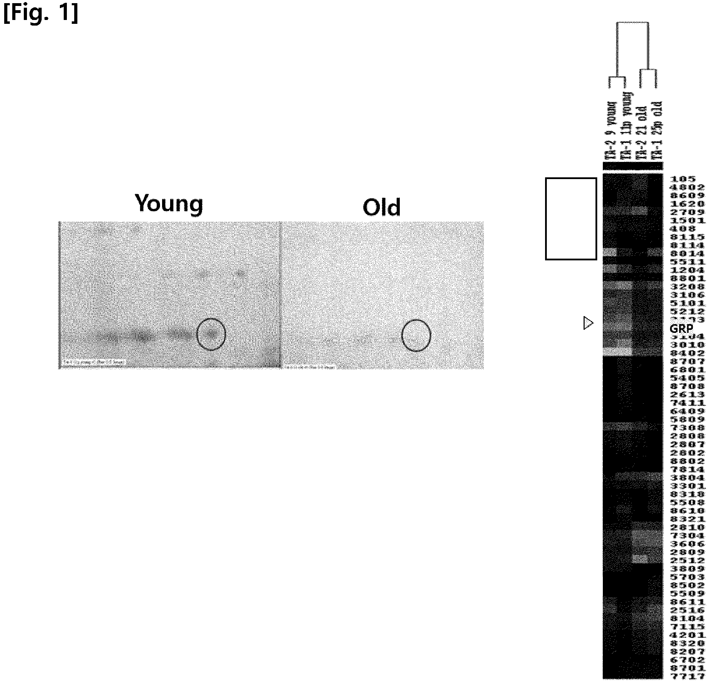

FIG. 1 is a list of proteins showing significant changes as a result of proteomic analysis performed on young cells and old cells.
FIG. 2 shows the peptide peaks identified by MALDI-TOF-TOF analysis of GRP78 protein which showed a tendency to significantly decrease in old cells.
FIG. 3 shows a wild-type peptide sequence, identified as a GRP78 recognition site by MALDI-TOF-TOF analysis, and variant peptide sequences thereof.
FIG. 4 shows peptide sequences identified as having phosphorylation and oxidation potentials among the full-length sequence of GRP78 protein by protein structure analysis and sequencing.
FIG. 5 shows peptide sequences that bind to nucleotides in the nucleus, among GRP78-derived peptides.
FIG. 6 shows peptide sequences that bind to other substrates, among GRP78-derived peptides.

### Detailed Description and Preferred Embodiments of the Invention

Unless otherwise defined, all the technical and scientific terms used in the present specification have the same meanings as commonly understood by those skilled in the art to which the present disclosure pertains. In general, the nomenclature used in the present specification is well known and commonly used in the art.

In the present invention, GRP78 (glucoseregulated protein 78) showing a significant expression change in old stem cells was identified by examining proteomic changes as senescence of adult stem cells progressed. It was confirmed that the expressions of GRP78 gene and protein in old stem cells significantly decreased compared to those in young highly efficient stem cells. Accordingly, the amino acid sequence of the GRP78 protein was analyzed by MALDI-TOF/TOF and a wild-type peptide sequence identified as a GRP78 recognition site and variant peptide sequence were obtained. Also, protein structure analysis and sequencing were performed and peptide sequences having phosphorylation and oxidation potentials among the full-length sequence of the GRP78 protein were obtained. In addition, using endoplasmic reticulum chaperone BiP protein, peptides for GRP78 screening, which bind to nucleotides in the nucleus, and peptides for GRP78 screening, which bind to other substrates, were synthesized.

The GRP78-derived peptide of the present invention can serve as a cell surface marker that binds to the binding domain of the GRP78 protein on the cell surface, thereby screening cells labeling GRP78. In addition, the effect of the GRP78 protein can be increased by treating cells, which have decreased expression of the GRP78 protein, with the GRP78-derived peptide, or introducing the GRP78-derived peptide into the cells.

Therefore, one aspect of the present specification is directed to a peptide for screening highly efficient stem cells or enhancing the efficiency of stem cells, the peptide being represented by an amino acid sequence selected from the group consisting of SEQ ID NOs: 1 to 18.

The peptide may be derived from GRP78.

The nucleotide sequence and amino acid sequence of GRP78 may be obtained from a known database such as NCBI's GenBank (e.g., GenBank Accession AAA52614.1).

The peptides represented by the amino acid sequences of SEQ ID NOs: 1 to 3 may recognize the 1-32 site of GRP78, and may be the 1-32 wild-type recognition site (WT): MEHRTRSWALGLSILGFFALLFSAGFVQQAHA (SEQ ID NO: 1), the 1-32 variant recognition site (KO1): MEHRNRSWALGLSILGFFALLFSAGFVQQAHA (SEQ ID NO: 2), and the 1-32 variant recognition site (KO2): MDHRNRSWALGLSILGFFALLFSAGFVQQAHA (SEQ ID NO: 3).

The peptides represented by the amino acid sequences of SEQ ID NOs: 4 to 6 preferably recognize the phosphorylation or oxidation site of the GRP78 protein, and may more preferably be 139-152 TFAPEEISAMVLTK (SEQ ID NO: 4), 186-197 DAGTIAGLNVMR (SEQ ID NO: 5), and 325-336 AKFEELNMDLFR (SEQ ID NO: 6).

The peptides represented by the amino acid sequences of SEQ ID NOs: 7 to 14 may be the 125-280 site of GRP78, may preferably recognize ATP in the nucleus, which is regulated by GRP78, and may more preferably be KPYIQVDIGGGQTKTFAPEEISA (SEQ ID NO: 7), MVLTKMKETAEAYLGKKVTH (SEQ ID NO: 8), AVVTVPAYFNDAQRQAT (SEQ ID NO: 9), KDAGTIAGLNVMRIINEPTAAA (SEQ ID NO: 10), IAYGLDKREGEKNILVFDLGGG (SEQ ID NO: 11), TFDVSLLTIDNGVFEV (SEQ ID NO: 12), VATNGDTHLGGEDFDQRV (SEQ ID NO: 13), and MEHFIKLYKKKTGKDVRK (SEQ ID NO: 14).

Since GRP78 regulates homeostasis of ATP and calcium in the endoplasmic reticulum (ER) and ATP is a nucleotide, the peptides represented by the amino acid sequences of SEQ ID NOs: 7 to 14 are preferably peptides having a nucleotide-binding domain (NBD), and can increase the efficiency of stem cells by regulating the homeostasis of ATP and calcium in the ER.

The peptides represented by the amino acid sequences of SEQ ID NOs: 15 to 18 may be the 420-500 site of GRP78, preferably recognize other substrates that are ATP degradation products in the nucleus, which are regulated by GRP78, and may more preferably be CPLTLGIETVGGVMTKLIPR (SEQ ID NO: 15), NTVVPTKKSQIFSTASDNQP (SEQ ID NO: 16), TVTIKVYEGERPLTKDNHLL (SEQ ID NO: 17), and GTFDLTGIPPAPRGVPQIEVT (SEQ ID NO: 18).

Since GRP78 regulates homeostasis of ATP and calcium in the endoplasmic reticulum (ER) and stimulates ATP degradation, the peptides represented by the amino acid sequences of SEQ ID NOs: 15 to 18 are preferably peptides having a substrate-binding domain (SBD), and may bind to a substrate so as to be able to bind to ATP itselfs, thereby increasing the efficiency of stem cells.

The peptide of the present invention may be used to screen stem cells expressing the GRP78 protein on the cell surface. In addition, the peptide of the present invention may be introduced into the cytoplasm of stem cells in which intracellular expression of the GRP78 protein decreased due to senescence or external stimulation, thereby screening highly efficient stem cells and increasing stemness.

The peptide of the present invention may be easily produced by chemical synthesis methods known in the art (Creighton, Proteins; Structures and Molecular Principles, W H Freeman and Co, NY, 1983). Typical methods include, but are not limited to, liquid or solid phase synthesis, fragment condensation, and F-MOC or T-BOC chemistry (Chemical Approaches to the Synthesis of Peptides and Proteins, Williams et al, Eds., CRC Press, Boca Raton Florida, 1997; Chemistry of peptide synthesis, N. Leo Benoiton, Taylor & Francis, 2005, CRC press, page 140-143).

In addition, the peptide of the present invention may be produced by genetic engineering methods. First, a DNA sequence encoding the peptide is constructed according to a conventional method. The DNA sequence may be constructed by PCR amplification using suitable primers. Alternatively, the DNA sequence may also be synthesized by a standard method known in the art, for example, an automated DNA synthesizer (e.g., commercially available from Biosearch or Applied Biosystems). The constructed DNA sequence is inserted into a vector comprising one or more expression control sequences (for example, promoters, enhancers, etc.), which are operatively linked to the DNA sequence and regulate the expression of the DNA sequence, and a host cell is transformed with the resulting recombinant expression vector. The resulting transformant is cultured in suitable medium under suitable culture conditions so that the DNA sequence is expressed, and then a substantially pure peptide encoded by the DNA sequence is recovered from the cell culture. The recovery of peptide may be carried out by a conventional method known in the art (for example, chromatography). As used herein, the term "substantially pure peptide" means that the peptide is substantially free from any other proteins derived from the host. The genetic engineering method for synthesis of the peptide of the present invention can be found in the following literature: Maniatis et al, Molecular Cloning; A laboratory Manual, Cold Spring Harbor laboratory, 1982; Sambrook et al, Molecular Cloning: A Laboratory Manual, Cold Spring Harbor Press, NY, Second(1998) and Third(2000) Edition; and Hitzeman et al, J Biol Chem, 255:12073-12080, 1990.

In the present invention, the highly efficient stem cells may express GRP78.

The highly efficient stem cells may be non-senescent young stem cells.

The stem cells are preferably adult stem cells, but are not limited thereto.

As used herein, the term "stem cells" refers to pluripotent cells capable of differentiating into endodermal, mesodermal and ectodermal cells in animals, or multipotent cells capable of differentiating into closely related cells in tissues or functions. The term refers to a cell capable of dividing into two or more new cells while having self-renewal ability, and the stem cells may be classified into pluripotent stem cells, and multipotent stem cells.

As used herein, the term "adult stem cells" refers to multipotent cells obtained by isolating stem cells from each tissue and culturing the isolated stem cells *in vitro,* and includes bone marrow stem cells, retinal stem cells, retinal Muller glial cells, neural stem cells, and the like. The stem cells may be derived from mammals, including humans and primates, as well as livestock such as cattle, pigs, sheep, horses, dogs, rats, rats, and cats. Preferably, the stem cells may be derived from humans.

As used herein, the term "senescence" means that the differentiation or proliferation potential of adult stem cells decreases due to an increase in the age of an individual or an increase in the number of passages.

As used herein, the term "passage" means replacing a culture vessel or dividing and culturing a cell population, in a method in which a portion of cells are periodically transferred to a fresh culture vessel in order to continuously culture the cells in a healthy state for a long period of time, and then the cells are continuously subcultured while the culture medium is replaced. One passage refers to replacing the culture vessel once or dividing and culturing the cell population. In the present invention, low-passage or young-passage stem cells may refer to stem cells having a high differentiation ability or therapeutic effect, and highpassage or old-passage stem cells may refer to stem cells having a low differentiation ability or therapeutic effect, but are not limited thereto.

The young passage may have a passage number of 0 to 3, preferably 0 to 2. In addition, the old passage may have a passage number of 15 to 25, preferably 20 to 25.

The young stem cells may have a passage number of 0 to 3, preferably 0 to 2. In addition, the old stem cells may have a passage number of 15 to 25, preferably 20 to 25.

That is, the "old stem cells" refers to stem cells in which the differentiation or proliferation ability of adult stem cells has decreased due to an increase in the number of passages, and the "highly efficient stem cells" refers to stem cells that have excellent efficacy as cell therapy products because the adult stem cells therein have a very high differentiation or proliferation ability. In other words, the "highly efficient stem cells" refers to those that exhibit the differentiation or proliferation potential equivalent to young-passage stem cells.

Another aspect is directed to a method of screening highly efficient stem cells using a peptide represented by an amino acid sequence selected from the group consisting of SEQ ID NOs: 1 to 18.

The method may comprise steps of: (a) conjugating a fluorescent to a peptide specific for GRP78; (b) binding the fluorescent-conjugated peptide to stem cells; and (c) screening GRP78-expressing stem cells under a fluorescence microscope.

Still another aspect is directed to a composition for screening highly efficient stem cells, the composition comprising a peptide represented by an amino acid sequence selected from the group consisting of SEQ ID NOs: 1 to 18.

Yet another aspect is directed to a kit for screening highly efficient stem cells, the kit comprising a peptide represented by an amino acid sequence selected from the group consisting of SEQ ID NOs: 1 to 18.

The kit may be an RT-PCR kit, a DNA chip kit or a protein chip kit.

The kit may be a diagnostic kit composed of one or more compositions, solutions or instruments, which are suitable for analysis methods, and may be an RT-PCR kit, a DNA chip kit or a protein chip kit. The RT-PCR kit may comprise, in addition to each primer pair specific for the marker gene, a test tube or another suitable container, a reaction buffer, deoxynucleotides (dNTPs), enzymes such as Taqpolymerase, reverse transcriptase and DNase, an RNase inhibitor, DEPC-water, sterile water, etc. Also, it may comprise a primer pair specific to a gene which is used as a quantitative control. The DNA chip kit may comprise a substrate in which cDNA corresponding to the gene or a fragment thereof is attached as a probe. The substrate may contain cDNA corresponding to a quantification control gene or a fragment thereof.

In addition, the kit may be a diagnostic kit comprising an agent for measuring the protein level, in which the agent for measuring the protein level is preferably an antibody specific for the protein. Thus, the kit comprising the agent for measuring the protein level may be a kit for detection of markers, which comprises essential elements required for carrying out, for example, ELISA. This kit may also comprise reagents that may detect antibodies forming "antigen-antibody complexes", for example, labeled secondary antibodies, chromophores, enzymes (e.g., conjugated with antibodies) and their substrates. Also, it may comprise an antibody specific to a control protein for quantification.

Furthermore, the amount of antigen-antibody complexes formed may be quantitatively determined by measuring the signal intensity of a detection label. Such a detection label may be selected from the group consisting of, but not necessarily limited to, enzymes, fluorescent substances, ligands, luminescent substances, microparticles, redox molecules and radioactive isotopes.

Methods for measuring protein levels include, but not necessarily limited to, Western blotting, ELISA, radioimmunoassay, radio immunodiffusion, Ouchterlony immunodiffusion assay, rocket immunoelectrophoresis assay, immunohistostaining assay, immunoprecipitation assay, complement fixation assay, fluorescence activated cell sorter (FACS), protein chip assay, etc., and the protein level may be measured using methods known to those skilled in the art.

Yet another aspect is directed to a method of increasing the efficiency of stem cells by treating the stem cells with a peptide represented by an amino acid selected from the group consisting of SEQ ID NOs: 1 to 18, or introducing the peptide into the stem cell.

The stem cells having increased efficiency may express GRP78.

The stem cells are preferably adult stem cells, but are not limited thereto.

As used herein, the term "stem cells" refers to pluripotent cells capable of differentiating into endodermal, mesodermal and ectodermal cells in animals, or multipotent cells capable of differentiating into closely related cells in tissues or functions. The term refers to a cell capable of dividing into two or more new cells while having self-renewal ability, and the stem cells may be classified into pluripotent stem cells, and multipotent stem cells.

As used herein, the term "adult stem cells" refers to multipotent cells obtained by isolating stem cells from each tissue and culturing the isolated stem cells *in vitro,* and includes bone marrow stem cells, retinal stem cells, retinal Muller glial cells, neural stem cells, and the like. The stem cells may be derived from mammals, including humans and primates, as well as livestock such as cattle, pigs, sheep, horses, dogs, mouse, rats, and cats. Preferably, the stem cells may be derived from humans.

As used herein, the term "increasing the efficiency" of stem cells means that the differentiation or proliferation potential of adult stem cells increases. The term "highly efficient stem cells" or "stem cells having increased efficiency" refers to those that exhibit the differentiation or proliferation potential equivalent to young-passage stem cells.

As described herein, "treating with the peptide" or "introducing the peptide into" may be performed by treating a cell culture medium directly with the peptide or treating the cell culture medium with a mixture of the peptide and a biomaterial for culture, and the biomaterial refers to a synthetic polymer or a natural polymer.

The synthetic polymer is preferably selected from among poloxamer, polyethylene glycol and polypropylene glycol, and the natural polymer is preferably selected from among vitronectin, collagen, gelatin, alginic acid, chondroitin sulfate, fibronectin and extracellular matrix protein.

Yet another aspect is directed to a composition for increasing efficiency of stem cells, the composition comprising a peptide represented by an amino acid sequence selected from the group consisting of SEQ ID NOs: 1 to 18.

### Examples

Hereinafter, the present invention will be described in more detail with reference to examples. It will be obvious to skilled in the art that these examples are merely to illustrate the present invention, and the scope of the present invention is not limited by these examples.

### Example 1: Change and Identification of GRP78 Protein by Proteomic Analysis

In order to examine the changes in proteomics with the progress of senescence of adult stem cells, proteomic analysis was performed using each cell pellet cultured at each passage.

### (1) Protein Extraction (Protein Sample Preparation)

Protein extraction from a sample was performed as follows. A 2DE lysis solution composed of 7M urea, 2M thiourea, 4% (w/v) 3-[(3-cholamidopropyl)dimethylammonio]-1-propanesulfonate (CHAPS), 1% (w/v) dithiothreitol (DTT), 2% (v/v) Pharmalyte and 1 mM benzamidine was added to the sample. For protein extraction, the mixture was vortexed for 1 hour and centrifuged at 12,000 rpm and 25°C for 1 hour. The supernatant was used as a sample for two-dimensional electrophoresis. Measurement of protein concentration was performed by the Bradford method.

### (2) 2D Electrophoresis

For primary isoelectric focusing (IEF), IPG strips were reswollen in a reswelling buffer (containing 7M urea, 2M thiourea, 2% 3-[(3-cholamidopropy)dimethyammonio]-1-propanesulfonate (CHAPS), 1% dithiothreitol (DTT), and 1% Pharmalyte) at room temperature for about 12 to 16 hours. 100 µg of the sample per strip was used, and IEF was performed at 20°C using the Multiphore II system (Amersham Biosciences) according to the manufacturer's manual. For IEF, the voltage was increased from 150V to 3,500V within 3 hours and maintained at 3,500V for 26 hours, and IEF was completed at 96 kVh.

Before SDS-PAGE was performed, IPG strips were incubated in a 1% DTT-containing equilibration buffer (50 mM Tris-Cl, pH 6.8, 6M urea, 2% SDS, and 30% glycerol) for 10 minutes, and immediately further incubated in a 2.5% iodoacetamide-containing equilibration buffer for 10 minutes. Equilibrated strips were arranged on SDS-PAGE gels (20×24 cm, 10 to 16%), and run at 1.7 kVh and 20°C using a Hoefer DALT 2D system. After 2D electrophoresis was completed, proteins on the 2D gels were visualized by silver staining according to the method described in Oakley et al., Anal. Biochem. 1980, 105:361-363, and a glutaraldehyde treatment step was omitted for protein identification by mass spectrometry. The silver-stained 2D gel was scanned, and stored as a TIFF file.

### (3) Image Analysis

Quantitative analysis for examining changes in expression of protein spots from the scanned image was performed using PDQuest software (version 7.0, BioRad). The quantity of each spot was normalized by the total valid spot intensity, and protein spots showing at least 2-fold significant changes in expression compared to control were selected.

### Example 2: Amino Acid Sequencing of GRP78 Protein Obtained by MALDI-TOF/TOF Analysis

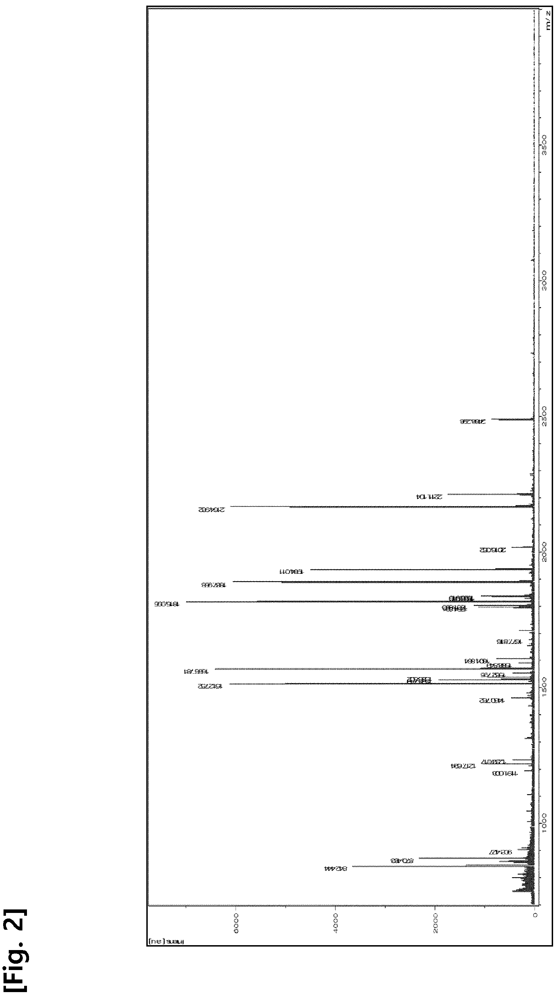

The protein spot selected in the 2DE analysis was washed with 50% acetonitrile, dried under vacuum, and then subjected to in-gel digestion with trypsin. The degraded sample was analyzed using a BRUKER AUTOFLEX SPEED with LIFTTM ion optics. The results of the mass spectrometry sequencing of the peptide were provided in the MASCOT search form (FIG. 2).

### Example 3: GRP78-Specific Recognition Related Sequences

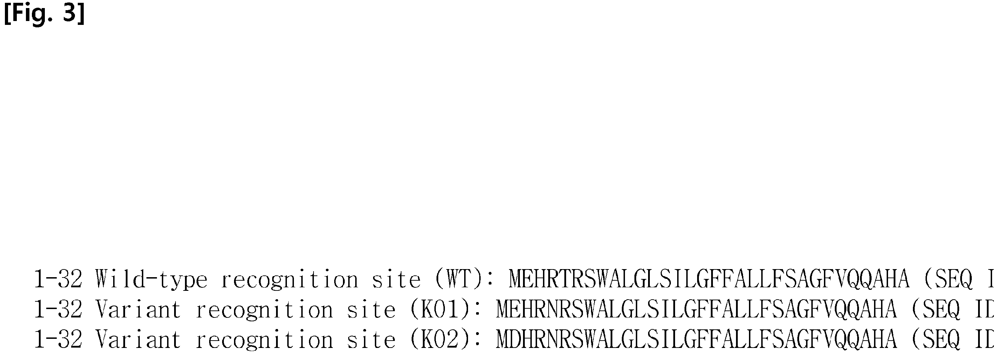

FIG. 3 shows a wild-type peptide sequence, identified as a GRP78 recognition site by MALDI-TOF-TOF analysis, and variant peptide sequences thereof, developed by the present inventors.
1-32 wild-type recognition site (WT): MEHRTRSWALGLSILGFFALLFSAGFVQQAHA (SEQ ID NO: 1)
1-32 variant recognition site (KO1): MEHRNRSWALGLSILGFFALLFSAGFVQQAHA (SEQ ID NO: 2)
1-32 variant recognition site (KO2): MDHRNRSWALGLSILGFFALLFSAGFVQQAHA (SEQ ID NO: 3)

The sequence consisting of 1^{st} to 32^{nd} amino acids of the GRP78 protein was selected as a wild-type sequence, and variant sequences were selected.

### Example 4: Phosphorylation Sequence

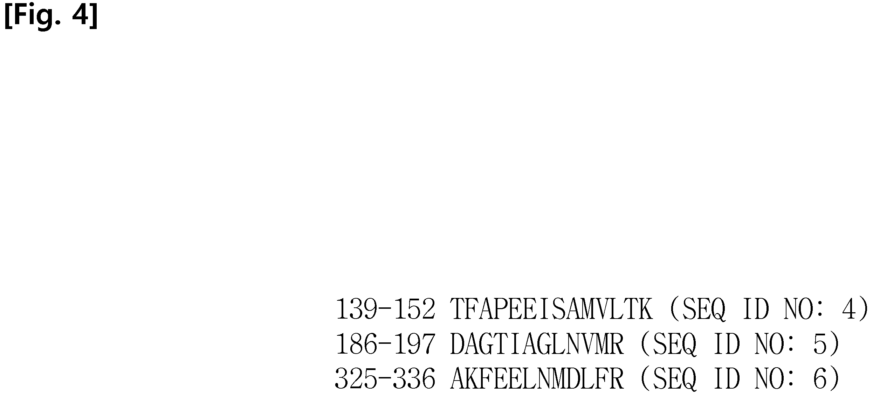

FIG. 4 shows peptide sequences identified as having phosphorylation and oxidation potentials among the full-length sequence of GRP78 protein by protein structure analysis and sequencing. In this Example, sites predicted to have the binding domain or a major functional domain in the GRP78 protein were selected and synthesized.
139-152 TFAPEEISAMVLTK (SEQ ID NO: 4)
186-197 DAGTIAGLNVMR (SEQ ID NO: 5)
325-336 AKFEELNMDLFR (SEQ ID NO: 6)

### Example 5: GRP78 Peptide Synthesis

To synthesize peptides for GRP78 screening, the endoplasmic reticulum chaperone BiP protein [Homo sapiens] (NP_005338.1) (SEQ ID NO: 19), which consists of 654 amino acids, was used. In addition to this sequence, various sequences including the GRP78 binding domain WIFPWIQL (SEQ ID NO: 20) may be used. Other detailed information is available from https://www.uniprot.org/uniprot/P11021.

Peptides for GRP78 screening were synthesized in large amounts using a peptide synthesizer (Apex 396, AAPPTec, Louisville, KY, USA). The synthesized peptides were purified by reverse phase high-performance chromatography (HPLC) using a Vydac C18 column and a 0.1% TFA-containing water/acetonitrile gradient. The synthesized peptides having a purity of 98% or higher were used. The synthesized peptides were freeze-dried, and then stored in dark at -80°C until use.

### (1) Peptides That Bind to Nucleotides in Nucleus

To synthesize peptides for GRP78 screening that bind to nucleotides in the nucleus, nucleotide or amino acid sequences containing the 125-280 sequence of GRP78, or those containing the following sequences were selected.

As a proteins in the structure of GRP78, NBD (nucleotide binding domain) is divided into two large spherical domains (I and II), and each of the domains is subdivided into subdomains A and B. That is, NBD is divided into IA, IB, IIA and IIB sequences. SEQ ID NOs: 7 and 8 contain IA, SEQ ID NOs: 9 and 10 contain IB, SEQ ID NOs: 11 and 12 contains IIA, and SEQ ID NOs: 13 and 14 contain IIB. The peptides recognize and bind to ATP (nucleotide) which is regulated by GRP78 in cells.

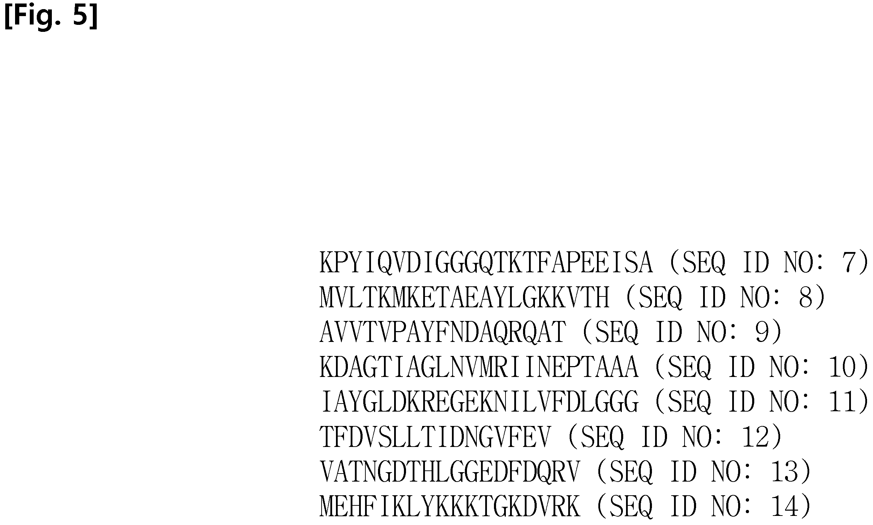

The eight sequences are as follows and are also shown in FIG. 5.
KPYIQVDIGGGQTKTFAPEEISA (SEQ ID NO: 7)
MVLTKMKETAEAYLGKKVTH (SEQ ID NO: 8)
AVVTVPAYFNDAQRQAT (SEQ ID NO: 9)
KDAGTIAGLNVMRIINEPTAAA (SEQ ID NO: 10)
IAYGLDKREGEKNILVFDLGGG (SEQ ID NO: 11)
TFDVSLLTIDNGVFEV (SEQ ID NO: 12)
VATNGDTHLGGEDFDQRV (SEQ ID NO: 13)
MEHFIKLYKKKTGKDVRK (SEQ ID NO: 14)

The peptides of SEQ ID NOs: 7 and 8 were synthesized starting from the N-terminus by F-moc solid phase peptide synthesis. The synthesized peptide sequences were cut from resin, washed, freeze-dried, and then separated and purified by liquid chromatography. The molecular weights of the purified peptides were analyzed by MALDI-TOF analysis.

### (2) Peptides That Bind to Other Substrates

To synthesize peptides for GRP78 screening that bind to other substrates, sequences containing the 420-500 sequence of GRP78 or those containing the following sequences were selected.

After ATP binds to the NBD of GRP78 during ATP degradation, a change in the structure of the SBD (substrate binding domain) of the GRP78 sequence occurs to promote the degradation process occurring in the NBD. The SBD is divided into two domains: SBD alpha, and SBD beta. The two domains are represented by SEQ ID NOs: 15 and 16, and SEQ ID NOs: 17 and 18, respectively. These peptides bind to substrates that bind to degradation products produced by the degradation of ATP that is regulated by GRP78. These peptides may further promote the degradation process.

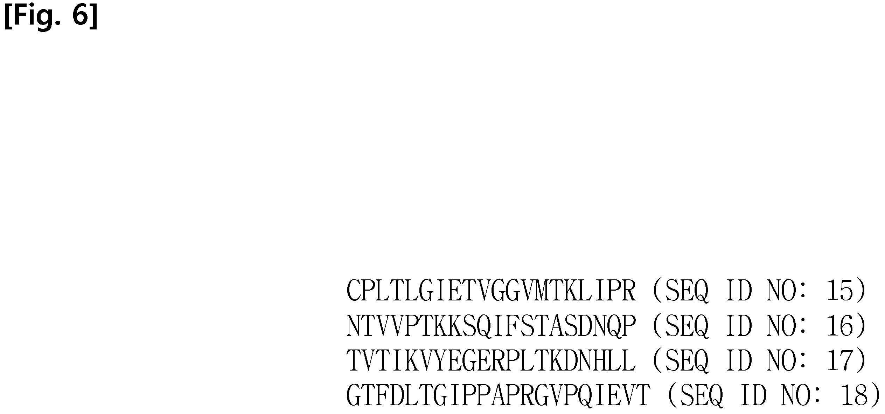

The four sequences are as follows and are shown in FIG. 6.
CPLTLGIETVGGVMTKLIPR (SEQ ID NO: 15)
NTVVPTKKSQIFSTASDNQP (SEQ ID NO: 16)
TVTIKVYEGERPLTKDNHLL (SEQ ID NO: 17)
GTFDLTGIPPAPRGVPQIEVT (SEQ ID NO: 18)

The peptides of SEQ ID NOs: 15 to 18 were synthesized by F-moc solid phase peptide synthesis. The synthesized peptide sequences were cut from resin, washed, freeze-dried, and then separated and purified by liquid chromatography. The molecular weights of the purified peptides were analyzed by MALDI-TOF analysis.

### Example 6: Screening of Stem Cells Using Synthetic GRP78 Peptides

To screen highly efficient stem cells using the peptides synthesized in Examples 3 to 5, fluorescent-labeled GRP78 peptides were used. That is, cells expressing GRP78 on the cell surface were screened. Alternatively, the GRP78 protein in the nucleus or other intracellular organelle was labeled with fluorescent to distinguish the GRP78 labeled in the nucleus from the one in the membrane. The cell permeability and change in intracellular distribution of each of the peptides were evaluated.

To evaluate the cell permeability of each of the fluorescent-labeled peptides, cells were cultured in a 12-well plate in a 5% CO₂ humidified chamber at 37°C and attached, and then the culture medium was removed. Each of the peptides was added to the cells at an optimal concentration, and control cells were treated with PBS. After culturing in a 5% CO₂ humidified chamber at 37°C for 2 hours and 8 hours, the cells were carefully washed three times with PBS, and were incubated with DAPI for 30 minutes, and the distribution of the peptide in the nucleus was observed. After incubation, the cells were washed twice with PBS and analyzed by fluorescence imaging with a fluorescence microscope (OLYMPUS IX-70).

### Industrial Applicability

According to the present invention, the GRP78-derived peptide comprising only a specific amino acid sequence capable of recognizing highly efficient stem cells, among the amino acid sequence of GRP78, makes it possible to screen only non-senescent young stem cells. In addition, when stem cells are treated with the GRP78-derived peptide or the GRP78-derived peptide is introduced into stem cells, the efficiency of the stem cells can be increased. Thus, the GRP78-derived peptide is useful for the production of stem cell therapy products having excellent efficacy.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims

### Sequence List Text

Electronic file attached

## Claims

1. A peptide for screening highly efficient stem cells, the peptide consisting of the amino acid sequence of SEQ ID NO: 8.

2. The peptide of claim 1, wherein the peptide is derived from GRP78.

3. The peptide of claim 1, wherein the peptide recognizes ATP in the nucleus, which is regulated by GRP78.

4. The peptide of claim 1, wherein the highly efficient stem cells express GRP78.

5. A method of screening highly efficient stem cells using the peptide of any one of claims 1 to 4.

6. The method of claim 5, comprising steps of:
(a) conjugating a fluorescent substance to a peptide specific for GRP78;
(b) binding the fluorescent-conjugated peptide to stem cells; and
(c) screening GRP78-expressing stem cells under a fluorescence microscope.

7. A kit for screening highly efficient stem cells comprising the peptide of any one of claims 1 to 4.

## Patentansprüche

1. Peptid zum Screening hocheffizienter Stammzellen, wobei das Peptid aus der Aminosäuresequenz von SEQ ID NO: 8 besteht.

2. Peptid nach Anspruch 1, wobei das Peptid von GRP78 abgeleitet ist.

3. Peptid nach Anspruch 1, wobei das Peptid ATP im Zellkern erkennt, das durch GRP78 reguliert wird.

4. Peptid nach Anspruch 1, wobei die hocheffizienten Stammzellen GRP78 exprimieren.

5. Verfahren zum Screening hocheffizienter Stammzellen unter Verwendung des Peptids nach einem der Ansprüche 1 bis 4.

6. Verfahren nach Anspruch 5, umfassend die folgenden Schritte:
(a) Konjugieren einer fluoreszierenden Substanz an ein für GRP78 spezifisches Peptid;
(b) Binden des fluoreszenzkonjugierten Peptids an Stammzellen; und
(c) Screening von GRP78-exprimierenden Stammzellen unter einem Fluoreszenzmikroskop.

7. Kit zum Screening hocheffizienter Stammzellen, umfassend das Peptid nach einem der Ansprüche 1 bis 4.

## Revendications

1. Peptide pour le criblage de cellules souches à haute efficacité, le peptide étant constitué de la séquence d'acides aminés de SEQ ID NO : 8.

2. Peptide selon la revendication 1, dans lequel le peptide est dérivé de GRP78.

3. Peptide selon la revendication 1, dans lequel le peptide reconnaît l'ATP dans le noyau, lequel est régulé par GRP78.

4. Peptide selon la revendication 1, dans lequel les cellules souches à haute efficacité expriment GRP78.

5. Procédé de criblage de cellules souches à haute efficacité utilisant le peptide selon l'une quelconque des revendications 1 à 4.

6. Procédé selon la revendication 5, comprenant les étapes consistant à :
(a) conjuguer une substance fluorescente à un peptide spécifique de GRP78 ;
(b) lier le peptide conjugué à la substance fluorescente à des cellules souches ; et
(c) cribler des cellules souches exprimant GRP78 au microscope à fluorescence.

7. Kit pour le criblage de cellules souches à haute efficacité comprenant le peptide selon l'une quelconque des revendications 1 à 4.
